# EUROPÄISCHE PATENTANMELDUNG

(11) **EP 0 933 069 A1**
(43) Veröffentlichungstag der Anmeldung: **04.08.1999**
(21) Anmeldenummer: 98810064.0
(22) Anmeldetag: 30.01.1998
(51) Int. Cl.: A61F 2/00

(54) **Hilfsmittel gegen Blasenschwäche oder Harninkontinenz**

(71) Anmelder: Tschannen, Peter, 3792 Saanen-Gstaad (CH)
(72) Erfinder: Tschannen, Peter, 3792 Saanen-Gstaad (CH)
(74) Vertreter: BOVARD AG - Patentanwälte

(57) **Zusammenfassung**

Das Hilfsmittel in Form eines Tampons dient der Unterdrückung von Blasenschwäche und Harninkontinenz bei Frauen. Der Tampon besteht aus zwei länglichen Tamponkörpern, die seitlich in einem Abstand in flexibler Weise verbunden sind. Zumindest im Bereich seiner Oberfläche besteht er vorzugsweise aus einem im Wesentlichen nicht absorptionsfähigen Material. Hierzu ist der gewebefreundliche Polyvinylalkohol geeignet. Der Tampon wirkt derart, dass durch seine Einführung in die Vagina ein deformierter Blasenhals eleviert wird, wobei der Schliessmuskelabschnitt der Blase wiederum seine normale Funktion ausführen und der Urin zurückgehalten werden kann.

## Beschreibung

Die vorliegende Erfindung betrifft ein mechanisches Hilfsmittel für die Blasenkontrolle, insbesondere ein Tampon für Frauen, die an Blasenschwäche oder Inkontinenz leiden. Durch die spezielle Form des Tampons wird bewirkt, dass bei seiner Anwendung eine Deformation des Blasenhalses so ausgeglichen wird, dass der Schliessmuskelabschnitt wiederum seine normale Verschlussfunktion ausüben kann.

Schon von einem Alter von ca. 45 Jahren an können Personen an leichter Inkontinenz leiden. Diese Erscheinung kann das Wohlbefinden der betroffenen Personen beträchtlich beeinträchtigen. Zum Auffangen des unbeabsichtigten Harnausflusses wurden bisher in solchen Fällen beispielsweise Wäscheeinlagen verwendet. Diese Wäscheeinlagen können jedoch nicht in jeder Situation verwendet werden und haben bei Sporttreibenden den Nachteil, dass sie auftragend sind.

Der Erfinder der vorliegenden Erfindung hat aus seiner Praxis als Frauenarzt die Erfahrung gewonnen, dass dieses Problem für eine beträchtliche Gruppe seiner Patientinnen von Bedeutung ist. Es wurde festgestellt, dass die Ursache der leichten Inkontinenz meistens mechanischer Natur ist und demzufolge durch eine mechanische Einwirkung behoben werden kann. Ursache ist nämlich ein deformierter Blasenhals, welcher durch eine Elevation in eine Position gebracht werden kann, in welcher die Schliessmuskulatur wiederum ihre normale Funktion ausüben kann. Es ist somit Aufgabe der vorliegenden Erfindung, ein Hilfsmittel zur Verfügung zu stellen, welches in der Lage ist, die leichte Inkontinenz von Frauen auf mechanische oder physikalische Weise zu beheben.

Es wurde gefunden, dass Probleme bei der Blasenkontrolle oder bei leichter Inkontinenz, an welcher viele Frauen leiden, durch die Einführung eines der Anatomie angepassten Tampons vermieden werden können. Durch eine Anwendung eines derartigen Hilfsmittels wird die Kontrolle des Harnausflusses bei Blasenschwäche in vielen Fällen erheblich verbessert. Versuche mit einfachen Tampons aus Material mit geringer Absorptionsfähigkeit zeigten positive Resultate. Sie hatten jedoch die Unzulänglichkeit, dass Tampons mit einer herkömmlichen Form bei gewissen Bewegungen zu einem Verrutschen neigten und dadurch ihre Stützwirkung nicht in jedem Moment gewährleistet war. Überraschenderweise konnte dieser Mangel behoben werden, indem Doppeltampons ( 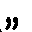 Twin-Tampons") gemäss der vorliegenden Erfindung verwendet wurden.

Gegenstand der vorliegenden Erfindung ist demzufolge ein Tampon gemäss der im Patentanspruch 1 gegebenen Definition.

Der erfindungsgemässe Tampon ist zweiteilig ausgestaltet und umfasst zwei in Längsrichtung flexibel verbundene Teilstücke. Vorzugsweise besteht der Tampon zumindest im Bereich seiner Oberfläche aus einem im Wesentlichen nicht absorptionsfähigen Material.

Durch die zweiteilige Form ( 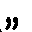 Twin-Tampon" oder 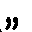 Zwilligstampon") wird im besonderen den anatomischen Gegebenheiten Rechnung getragen. Ein Verschieben des Tampons bei Bewegungen wird weitgehend ausgeschlossen, da die beiden miteinander verbundenen Tamponteile bei einem eingeführten Tampon gemäss der vorliegenden Erfindung links und rechts in der Vagina, wo genügend Raum vorhanden ist, positioniert sind. Dadurch kann die mechanische Stützwirkung in jeder Situation ausgeübt werden, was die Sicherheit erhöht.

Vorzugsweise besteht ein erfindungsgemässer Tampon aus zwei im Wesentlichen zylindrischen Tamponteilen, die entlang einer Mantellinie mit einem flexiblen Film, einer Folie oder einem andern Mittel in solcher Weise verbunden sind, dass sich der Abstand der beiden Teile entsprechend der anatomischen Bedingungen einstellen kann und die Bewegungsfreiheit nicht behindert wird.

Vorzugsweise besteht der Tampon mindestens an seiner Oberfläche aus Polyvinylalkohol PVA. Dieses Material ist gewebe- und hautfreundlich und weist nur eine geringe Absorptionsfähigkeit für wässrige Flüssigkeiten auf. Es wurde festgestellt, dass der Tampon für den vorgesehenen Zweck im Gegensatz zu normalen Tampons für die Monatshygiene keine Saugfähigkeit aufweisen muss, da die Funktion des Tampons nicht im Aufsaugen einer Flüssigkeit, sondern in einer mechanischen Druckausübung besteht. Die Absorptionsfähigkeit des Tampons für Flüssigkeiten soll dabei in Grenzen oder minimal gehalten werden, damit die natürliche Feuchtigkeit der Schleimhäute nicht beeinflusst wird.

Es hat sich gezeigt, dass sich das in der Chirurgie im Bereich von Operationen bewährte Material, nämlich Polyvinylalkohol, vorzüglich für die Oberfläche eines Tampons gemäss der vorliegenden Erfindung eignet. Der Tampon kann vollständig oder teilweise aus diesem Material bestehen. Selbstverständlich kommen ebenfalls andere Materialien in Frage, wie Polyurethan, Produkte aus Zellulose oder ähnlichen Materialien. Diese Materialien können derart ausgerüstet sein, dass die Absorptionsfähigkeit nach Wunsch begrenzt ist. Die Tamponteile können auch mit einer Schrumpffolien-Beschichtung, z.B. aus PE oder PP, versehen sein. Dadurch ist es möglich, den Tamponkörper aus einem kostengünstigen und leicht abbaubaren Material herzustellen.

Die Tamponteile weisen eine übliche Form auf und können den anatomischen Gegebenheiten angepasst werden. Sie können beispielsweise zylinder- oder ellipsoidförmig sein. Sie können auch Hohlräume aufweisen oder aber aus geschäumtem Material bestehen. Für die Einführung der Tampons gemäss der Erfindung ist ein üblicher Applikator vorgesehen, wobei die beiden Teile zwecks einer problemlosen Einführung zusammengepresst sind. Zu seiner Entfernung weist der Tampon einen Rückholfaden auf.

Anhand der beiliegenden Figuren wird nun die Erfindung beispielshaft erklärt. Es zeigt:
Fig. 1 einen Tampon gemäss der Erfindung in perspektivischer Ansicht.
Fig. 2 einen Twin-Tampon gemäss der Erfindung in einem röhrenförmigen Applikator
Fig. 3 einen Schnitt eines eingesetzten Twin-Tampons

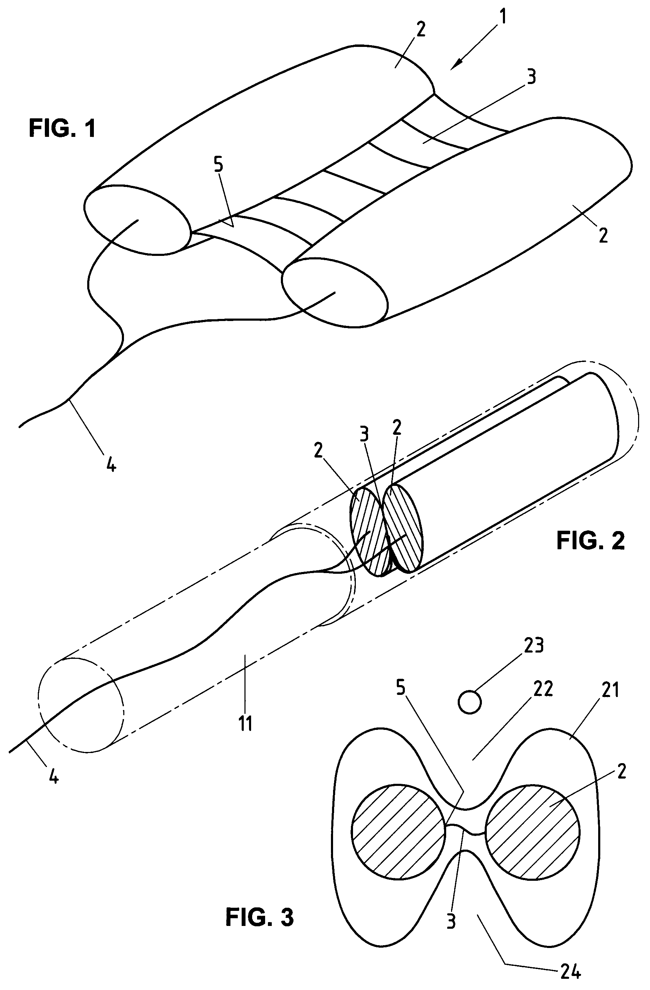

Gezeigt ist in Fig. 1 ein Tampon 1 aus einem geschäumten Polyvinylalkoholmaterial. Er besteht aus den beiden zylinderförmigen Teilen 2, die durch ein flächenförmiges Gebilde 3 mit Befestigungsnähten 5 vebunden sind. Am distalen Ende der Tamponteile ist ein Rückholfaden 4 an beiden Teilen 2 befestigt. Die Befestigung erfolgt normalerweise durch eine Verankerung des Fadens 4 innerhalb des Tamponkörpers.

Für die Applikation eines Twin-Tampons können übliche Einführhilfen zum Einsatz gelangen, wie sie ebenfalls bei Monatstampons verwendet werden. Fig. 2 zeigt eine perspektivische Ansicht eines Twin-Tampons 1 mit den Teilen 2 und dem Flächengebilde 3 in einem Röhrchen-Applikator 11. Ist der Tampon beim Einführen aus dem Röhrchen 11 ausgestossen, nimmt er seine vorgesehene Position ein, damit er seine Stützfunktion ausüben kann.

Fig. 3 zeigt schematisch einen Schnitt durch die Vagina mit einem eingesetzten Twin-Tampon, wobei die Bezugszeichen 22, 23 und 24 die Position der Blase, der Harnröhre bzw. des Darms zeigen. Die Tamponteile 2 befinden sich in einer beabstandeten Position entsprechend dem durch die Anatomie vorgegebenen Raum. In dieser Position besteht, sofern korrekt eingeführt, keine Gefahr eines Verschiebens der Tamponteile 2, so dass in jedem Fall der Tampon ununterbrochen die erwünschte Stützfunktion ausüben kann, die zu einer Elevation eines deformierten Blasenhalses führt, was die normale Funktion der Schliessmuskulatur erleichtert.

Die Form, Grösse und Deformierbarkeit der Tamponteile können entsprechend den anatomischen Voraussetzungen ausgestaltet sein, wobei verschiedene Grössen zur Verfügung gestellt werden können.

## Patentansprüche

1. Tampon für die Unterdrückung von Harninkontinenz bei Frauen, zur Einführung in die Vagina, damit ein deformierter Blasenhals eleviert wird, wodurch der Schliessmuskelabschnitt der Blase wiederum seine normale Funktion ausüben kann, dadurch gekennzeichnet, dass der Tampon aus zwei länglichen Tamponkörpern besteht, die seitlich in einem Abstand in flexibler Weise verbunden sind.

2. Tampon nach Patentanspruch 1, dadurch gekennzeichnet, dass die Tamponkörper zumindest im Bereich ihrer Oberfläche aus einem im Wesentlichen nicht absorbierfähigen Material bestehen.

3. Tampon nach Patentanspruch 2, dadurch gekennzeichnet, dass das im Wesentlichen nicht absorbierfähige Material Polyvinylalkohol PVA ist.

4. Tampon nach Patentanspruch 2, dadurch gekennzeichnet, dass der PVA verschäumt ist.

5. Tampon nach Patentanspruch 1, dadurch gekennzeichnet, dass die Tamponkörper aus Zellulose bestehen und im Bereich ihrer Oberfläche mit einem Folienmaterial aus Kunststoff überzogen sind.

6. Tampon nach einem der Ansprüche 1 bis 5, dadurch gekennzeichnet, dass die länglichen Tamponkörper zylinderförmig oder elipsoidförmig sind.

7. Tampon nach einem der Ansprüche 1 bis 6, dadurch gekennzeichnet, dass die Verbindung der beiden Tamponkörper aus einer Vielzahl von Fäden oder aus einem Flächengebilde besteht, derart, dass der Abstand der Tamponkörper maximal 2 cm beträgt.

8. Tampon nach einem der Ansprüche 1 bis 7, dadurch gekennzeichnet, dass er eine Einführhilfe und einen Rückholfaden aufweist.

9. Tampon nach einem der Ansprüche 1 bis 8, dadurch gekennzeichnet, dass die Tamponkörper eine Länge von 4-8 cm und einen Durchmesser von 1,5 - 3 cm aufweisen.
